# EUROPEAN PATENT SPECIFICATION

(11) **EP 2 449 994 B1**
(45) Date of publication and mention of the grant of the patent: **25.05.2016**
(21) Application number: 12151845.0
(22) Date of filing: 12.05.2006
(51) Int. Cl.: A61B 18/24, A61M 25/01, A61M 25/06, A61M 25/18, A61N 5/06, A61N 1/30

(54) **System with a light coupling adapter for photodynamic or photothermal therapy or photodynamic diagnosis**
System mit Lichtkopplungsadapter für photodynamische oder photothermische Therapie oder photodynamische Diagnose
Système comprenant un adaptateur de couplage de la lumière pour thérapie photodynamique ou photothermique ou diagnostic photodynamique

(30) Priority: 12.05.2005 SE 0501077; 13.05.2005 US 680779 P
(43) Date of publication of application: 09.05.2012
(62) Divisional of application: 06733490.4
(73) Proprietor: SpectraCure AB, 226 43 Lund (SE)
(72) Inventor: Svanberg, Sune, 224 65 Lund (SE)
(74) Representative: KIPA AB

(56) References cited:
- EP-A1- 0 549 097
- WO-A1-92/14515
- WO-A1-03/077000
- GB-A- 2 352 922
- US-A- 4 519 390
- US-A- 4 538 609
- US-A- 4 624 243
- US-A- 4 895 145
- US-A- 5 007 704
- US-A- 5 125 058
- US-A- 5 364 391
- US-A- 5 407 443

## Description

### FIELD OF THE INVENTION

The present invention relates to a system for interactive interstitial photodynamic or photothermal therapy and/or diagnosis of a tissue by an apparatus having at least one source of therapeutic or diagnostic light, such as a source of laser radiation, and at least one radiation conductor, such as a light guide in the form of an optical fibre, for transmitting light between said tissue and said apparatus, for instance the laser radiation to said tissue, and a light coupling adapter mechanism for conveniently connecting an optical path, including said optical fibre, between the tissue and the apparatus, for instance to said laser source.

### BACKGROUND OF THE INVENTION

A system and method for therapy and diagnosis using interactive interstitial photodynamic tumour therapy (PDT) and/or photothermal tumour therapy (PTT) and photodynamic diagnosis (PDD) is disclosed in WO 03/041575. The system comprises a plurality of optical fibres directly inserted at selected interstitial positions in tissue to be treated and/or diagnosed, such as cancer tissue.

In WO 03/041575 it is disclosed that for diagnosis, a source of diagnostic laser light is connected to one of the fibres, operating as a diagnostic transmission fibre for transmitting the diagnostic laser light towards the tissue. The remaining fibres are operating as receivers for light passing from the distal end of the diagnostic transmission fibre through the tissue to the distal ends of the remaining fibres and further on to detectors. Each fibre is used in sequence for being a transmitter and the other fibres being receivers. By calculations, a tomographic image of certain properties of the tissue may be obtained, such as fluorescence properties arising from certain sensitizers.

Further, WO 03/041575 discloses that, for therapy, the same system is used, whereby light is transmitted through the all fibres simultaneously to the tissue for activation of agents, such as sensitizers, which are localized to the tumour cells. Through the therapy, the cells are eradicated and the tissue goes into necrosis. Moreover, it is disclosed that the system may be used for photo-thermal therapy comprising transmitting radiation through the fibres, which heats the tissue sufficiently for killing the tumour cells. It may be noted that tumour cells may be more sensitive to heat than other cells.

In the diagnosis and therapeutic system of WO 03/041575, optical fibres are used, which are directly interstitially inserted into the tissue at places to be treated. The agent to be activated or sensitized may be brought to the tissue by systemic administration, intravenously or orally and accumulated in the relevant tissue by any mechanism. The surplus agent is normally removed by the body action, such as the liver or kidney, which may place a considerable burden on these organs or systems.

A further method of supplying the agent to a tissue would be the direct injection of the substance with a needle and syringe if the position of the tissue to be treated is well defined and known.

The agent may also be applied topically on the skin whereupon an iontophoretic technique could be used for improved transportation into the relevant tissue. Here, an electric current is generated by means of electrodes arranged in a suitable arrangement and the agent is transported with the current in dependence of the ionic properties of the agent.

In PDT/PTT and PDD, as mentioned above, light fibres are directly interstitially inserted in the tissue to be diagnosed/treated, i.e. the distal ends of the fibres, which proximally are connected to a distributor for radiation, are stuck into the tissue for interstitial arrangement therein. However, this has a number of drawbacks. For instance the fibres, or at least the distal ends thereof, have to be sterilized between subsequent treatments or patients. However, sterilization methods often deteriorate the optical qualities of the fibres, e.g. aggressive solvents when using plastic fibres or residues may be left on the surface of the fibres after sterilization or disinfection. Alternatively, the patient contaminated fibres have to be discarded, which is costly. When the distal fibre ends are inserted through the lumen of a cannula, which is stuck into the tissue to be treated/diagnosed, leaking is a concern that has to be dealt with. Furthermore, in case a drug has to be delivered to the tissue during treatment, e.g. in order to increase the amount of sensitizer in the tissue, it would be convenient, if the same access to the tissue, as used for the fibre, could be used. Today, the tissue is punctured with additional syringes, which may cause inconvenience or harm to the patient, e.g. due to an increased risk of infection and bleeding. Therefore, there is a need to provide a convenient way of interstitially accessing a tissue in a body for PDT/PTT and PDD, having a minimal number of access points by providing easy external access to the tissue when performing the treatment/diagnosis or between occasions of the same treatment/diagnosis. Moreover, a more cost-effective way of providing sterility between treatments or patients is desired.

Some attempts have been made to deal with these issues, which will be described below. However, hitherto none of these attempts has been successful.

EP 523417 discloses a system in which sleeves are arranged so that a radiating body may be inserted so that adjacent tissue is radiated and a sensor can be introduced in another sleeve to determine the effect of the radiation.

US 5,454,794 discloses a steerable light diffusion catheter. More specifically, a steerable catheter is disclosed which can treat luminal surfaces such as those occurring in the vascular tree, pulmonary tree, gastrointestinal tract, urological organs, etc. with Photodynamic Therapy (PDT) or other optical diffusing treatments. The catheter has a light-diffusing tip, which can be deflected allowing the catheter to be steered precisely. The light-diffusing tip on the steerable catheter is able to gain access to and enter the luminal system being treated. The catheter does not require a guidewire lumen for insertion, and thus the profile is reduced. A low profile device allows treatment light to be delivered to the walls of the most distal, small diameter lumen. However, US 5,454,794 is not suited for photodynamic diagnosis. Furthermore, the catheter of US 5,454,794 does not allow interstitial insertion thereof, only transluminal delivery. Transdermal insertion directly into a tumour, e.g. close to the skin, is not possible with this catheter. It is not suited for this purpose, partly due to its flexible design.

US 5,304,171 also discloses catheter devices and methods for delivering laser energy to a body. An apparatus for delivering laser energy to a site is disclosed that includes a flexible tube, a liquid, the tube having an opening in a first end through which the liquid can pass, means for providing a flow of the liquid into the tube, and a source of laser energy operationally associated with a second end of the tube, wherein the tube and the liquid are adapted to cooperate, when the tube contains the liquid, to conduct laser energy from the source and to emit a portion of the laser energy from the first end of the liquid-containing tube. According to a disclosed method, laser energy is conducted to the site by bringing the proximal end of the flexible tube near the site, filling at least a proximal portion of the tube with a liquid by introducing the liquid into the tube, allowing a portion of the liquid to flow out from the proximal end of the tube toward the site, and directing laser energy from a laser energy source into the distal end of the tube, whereby a portion of the laser energy emerges from the proximal end of the tube at the site. The liquid is for instance a radiographic contrast medium. The method is for instance used for removing an obstruction from a blood vessel in an animal.

The device of US 5,304,171 is neither suited for photodynamic diagnosis nor for photodynamic therapy. Furthermore, the catheter of US 5,304,171 does also not allow interstitial insertion thereof. Moreover, transdermal insertion directly into a tumour, e.g. close to the skin, is not possible with this catheter.

EP 1334748 discloses an apparatus for photodynamic therapy. Light generated by an implantable probe is used to illuminate a treatment site that has been perfused with a photoreactive agent. A number of different embodiments of implantable probes are disclosed. Preferably, an array of light emitting diodes (LEDs) or solid-state laser diodes (LDs) are mounted on a light bar inside the implantable probe and are energized either using a storage battery power source, an inductively coupled external transformer, or with current provided in leads running through a flexible catheter that extends outside the patient's body to an external source. The implantable probe is normally intended to be disposed inside a patient's body during a surgical procedure, at a treatment site, and left in place for several days (or longer) after an incision is closed, while light produced by the array of LEDs or solid-state LDs irradiates the treatment site. The apparatus of EP 1334748 is not configured for photodynamic diagnosis. Furthermore, interstitial insertion as well as removal of a probe has to be performed by a surgical procedure. Easy delivery of light to a tumour during short-term diagnosis and treatment is not provided.

WO2004/012589 discloses a catheter and method for diagnosis and treatment of diseased vessels. A catheter for detecting and treating diseased tissue in a blood vessel or other hollow body organ is provided. The catheter comprises an elongated tubular catheter shaft having a distal end comprising a light transmission zone. A first fiber lumen in the catheter shaft contains a diagnostic optical fiber having a distal end terminating within the light transmission zone for emitting and receiving light through the light transmission zone. A diagnostic subassembly at the proximal end and in communication with the diagnostic optical fiber processes diagnostic light for use in connection with a diagnostic method for detecting diseased tissue. A second fiber lumen can be provided in the catheter shaft for containing a treatment optical fiber for delivering treatment light from a light source at the proximal end of the catheter shaft to the light transmission zone. The treatment optical fiber has a distal end terminating within the light transmission zone for emitting light for treatment of the diseased tissue. An occlusion balloon is positioned on the distal end of the catheter shaft adjacent to the light transmission zone and in fluid communication with an inflation lumen. One or more infusion ports formed on or near the light transmission zone and in fluid communication with an infusion lumen deliver infusion fluid to the hollow body organ.

The catheter of WO2004/012589 does not allow interstitial insertion thereof. Moreover, transdermal insertion directly into a tumour, e.g. close to the skin, is not possible with this catheter.

The above described catheter devices are definitively not suited for a system involving a plurality of light guides inserted into a tissue, such as the system disclosed in WO03/041575 of same applicant, already mentioned above. WO03/041575 discloses a system and method for interactive, interstitial photodynamic and/or photothermal tumour therapy. The system comprises a distributor for distribution of radiation from at least one radiation source to a reaction site, or from the reaction site to at least one radiation sensor. A plurality of first radiation conductors are arranged for conduction of radiation to and from the reaction site and a plurality of second radiation conductors are arranged for emitting radiation from the radiation source and/or conduction of radiation to the radiation sensor. The distributor comprises two plane discs mounted abutting each other, one being fixed and the other turnable relative the first, each disc having holes arranged equally separated on a circle line the number of holes in the turnable disc being a multiple of the number of holes in the fixed disc. One end of the first radiation conductors are fixed in the holes of the fixed disc and one end of the second radiation conductors are fixed in the holes of the turnable disc.

Hence, there is a need for an improved device and method that provides a more advantageous way of accessing a tissue for interactive interstitial photodynamic or photothermal therapy and/or diagnosis of the tissue, and in particular allowing for increased flexibility, cost-effectiveness, or user friendliness.

### SUMMARY OF THE INVENTION

The closest prior art is formed by document US4519390. The invention and its advantageous embodiments are defined in the appended patent claims.

An object of the invention is to provide a system for conveniently arranging at least one radiation conductor for photodynamic or photothermal therapy and/or diagnosis.

An exemplary system for photodynamic or photothermal therapy and/or diagnosis of a tissue by an apparatus comprises at least one source of laser radiation, at least one radiation conductor for transmitting the laser radiation to said tissue, and a mechanism for connecting said radiation conductor to said laser source. The system comprises: an arranging device for arranging said radiation conductor from said tissue to said mechanism, said arranging device enclosing at least a portion of said radiation conductor. The arranging device may comprise a sleeve arranged from the tissue to said mechanism whereby said radiation conductor is inserteable in said sleeve after arranging said sleeve in said tissue. The system may further comprise a supply device for supplying an agent via said arranging device to said tissue. The sleeve may be provided with a port for supply of said agent. The supply device may be a syringe. A first sleeve may be arranged from the tissue to a distribution disk of said mechanism, and a second sleeve may be arranged from said distribution disk to said laser source, said second sleeve being provided with a port for the supply of said agent. The system may further comprise a pad including said agent and arranged at the skin adjacent said tissue and forming a first electrode connected to a first electric potential, whereby a second electric potential is connected to an electrically conductive lining arranged on at least one radiation conductor, to form a current for introduction of said agent into said tissue by iontophoresis. The conductive lining may be of a metal, such as silver.

An exemplary method in photodynamic or photothermal therapy and/or photodynamic diagnosis of a tissue by an apparatus having: at least one source of laser radiation, at least one radiation conductor for transmitting the laser radiation to said tissue, and a mechanism for connecting said radiation conductor to said laser source. The method may comprise: placing an arranging device in the tissue with a distal end in or close to a tissue to be treated; inserting a radiation conductor in the arranging device; and connecting the radiation conductor with said apparatus. The method may further comprise supplying an agent to said tissue to be treated via said arranging device.

According to a first aspect of the invention, a proximal end adapter for a catheter is provided. The adapter comprises: an adapter body, having proximal and distal ends, defining an axial lumen extending between the proximal and distal ends, a catheter mounting element at the distal end of the adapter body for sealingly mounting a catheter to the body; an optical connector mounting element at the proximal end for mounting an optical connector to the adapter body; and a light guide having proximal and distal ends, wherein said light guide is sealingly contained in said axial lumen defining an axial optical path extending between the proximal and distal ends of the adapter body. The proximal end adapter is configured for use in a system for interactive interstitial photodynamic or photothermal therapy and/or diagnosis of a tissue.

The optical connector mounting element at the proximal end for mounting an optical connector to the adapter body may be configured to directly mount to said adapter body.

The catheter mounting element at the distal end of the adapter body may comprise a mounting surface to sealingly engage said optical connector. The mounting surface may be an internal tapered surface sized to matingly and sealingly engage an external tapered surface of a Luer lock connector comprised in said optical connector. The mounting surface may be threaded for matingly and sealingly engage a threaded surface comprised in said optical connector.

The proximal end adapter according may comprise a radially compressible seal having a central bore receiving the light guide therein, positioned within the adapter body along the axial lumen at a distance from the proximal end, wherein the seal is radially outward compressed by said light guide to provide said sealing.

The light guide may be sealingly attached in said axial lumen by a fastening means, such as glue.

According to an embodiment, the catheter is a transcutaneous intravenous catheter device. The catheter may have a relatively short length of approximately less than 10 cm.

The adapter, when mounted to said catheter, seals said lumen against fluid connection from said proximal side of said adapter and provides light connection from said proximal side of said adapter to said lumen via said light guide.

According to a further aspect of the invention, a combination of the above-mentioned adapter and a hollow catheter is provided. The catheter mounting element is adapted to connect the adapter body to the hollow catheter so that the hollow catheter extends from the distal end of the adapter body.

The proximal end of the light guide may be positioned within the adapter body at a distance from the proximal end of the adapter body, and wherein the distal end of the light guide extends from the distal end of said adapter body into the hollow catheter.

The hollow catheter may define an axially extending catheter lumen, wherein the light guide is radially in contact with the interior wall of the axially extending catheter lumen.

The hollow catheter may define an axially extending catheter lumen, wherein the light guide has a diameter less than the interior diameter of the catheter lumen, so that a coaxial, radially offset, channel is formed in the catheter lumen.

The secondary lumen may include an entrance port opening into a portion of the axial lumen of the catheter body.

The combination may further comprise a needle that is inserteable and retractable in the hollow catheter for arranging said catheter to said tissue when said adapter is disconnected from said catheter, such that said radiation fibre is inserteable through the catheter or the needle towards said tissue.

According to yet a further aspect of the invention, a system configured for photodynamic or photothermal therapy and/or photodynamic diagnosis of a tissue is provided. The system comprises in combination an assembly of: a proximal end adapter for a catheter comprising an adapter body, having proximal and distal ends, defining an axial lumen extending between the proximal and distal ends, a catheter mounting element at the distal end of the adapter body for sealingly mounting a catheter to the adapter body, and a hollow catheter, wherein the catheter mounting element connects a catheter body to the hollow catheter so that the hollow catheter extends from the distal end of the catheter body; a first light guide having proximal and distal ends, wherein said first light guide is sealingly contained in said axial lumen defining an axial optical path extending between the proximal and distal ends of the adapter body towards said tissue and into close proximity thereof or interstitially into said tissue; an optical connector mounting element at the proximal end having mounted an optical connector to the adapter body, and wherein said optical connector is coupled to a light source via a second light guide, such that light from said light source is transmittable from said light source via said second light guide and further via said optical connector to said first light guide via said proximal end adapter towards said tissue through said catheter.

The first light guide may be inserteable into said catheter after arranging said sleeve in said tissue.

The system may further comprise a supply device for supplying an agent via said catheter to said tissue.

The catheter may be provided with a port for supply of said agent.

The system may further comprise a pad including said agent and arranged at the skin adjacent said tissue and forming a first electrode connected to a first electric potential, whereby a second electric potential is connected to an electrically conductive lining arranged on at least one of said radiation fibres, to form a current for introduction of said agent into said tissue by iontophoresis. The conductive lining may be of a metal, such as silver.

The system may further comprise a second adapter for releasably connecting the proximal end of said adapter body to a further optical connector proximally arranged at said optical connector, in order to ensure patient safety and to avoid contamination of re-usable articles of said system.

According to another aspect, a method for use of a proximal end adapter of a catheter is provided. The adapter has an adapter body, having proximal and distal ends, defining an axial lumen extending between the proximal and distal ends, and a catheter mounting element at the distal end of the adapter body for sealingly mounting a catheter to the body; an optical connector mounting element at the proximal end for mounting an optical connector to the adapter body; and a light guide having proximal and distal ends, wherein said light guide is sealingly contained in said axial lumen defining an axial optical path extending between the proximal and distal ends of the adapter body. The method comprises: mounting the adapter to a catheter housing by said catheter mounting element, to provide the light guide in a lumen of the catheter; mounting an optical connector to said optical connector mounting element of said adapter, thus providing an optical path via said adapter.

The mounting of the adapter and the mounting of the optical connector may comprise threadably mounting thereof.

According to a further aspect, a method for photodynamic or photothermal therapy and/or diagnosis of a tissue is provided. The method comprises providing at least one source of laser radiation, and providing at least one radiation fibre for transmitting the laser radiation to said tissue, arranging a catheter interstitially in said tissue; mounting a proximal end adapter to the proximal end of the catheter, said adapter having a radiation fibre thus mounted in a lumen of the catheter to said tissue, connecting said adapter to a distal radiation fibre via an optical connector coupled to said at least one source of laser radiation, such that when said adapter is connected to said connector laser radiation from said at least one source of laser radiation is transmitted via said distal optical fibre to said at least one radiation fibre and said tissue.

The method may, prior to mounting the adapter to the catheter, comprise inserting a needle into the tissue, said needle having a catheter sleeve arranged over said needle, removing said needle, and inserting at least one radiation fibre in said sleeve.

The method may comprise retracting of said a proximal end adapter from said lumen, draining said tissue from liquid through said lumen, and re-mounting the proximal end adapter to the proximal end of the catheter.

According to a further aspect, a use of an intravenous access device for interstitially coupling light to a tissue is provided.

### BRIEF DESCRIPTION OF DRAWINGS

Further objects, features and advantages of the invention will appear from the following detailed description of several embodiments of the invention with reference to the drawings, in which:
Fig. 1 is a schematic view of a previously known system in which the invention may be used;
Fig. 2 is a schematic view of the system of Fig. 1 adapted for diagnosis;
Fig. 3 is a cross-sectional view of a sleeve for an optical fibre;
Fig. 4 is a schematic view of an embodiment of the invention;
Fig. 5 is a schematic view of another embodiment of the invention;
Fig. 6 is an enlarged view of a portion of Fig. 5;
Fig. 7 is a schematic plane view of a conventional transcutaneous intravenous catheter device,
Fig. 8a is a cross sectional view of a proximal end adapter for a catheter;
Fig. 8b is a cross sectional view of another proximal end adapter; and
Figs. 9a, 9b, 10a, 10b and 11-16 are cross-sectional views of various proximal end adapter and catheter assemblies.

### DETAILED DESCRIPTION OF EMBODIMENTS

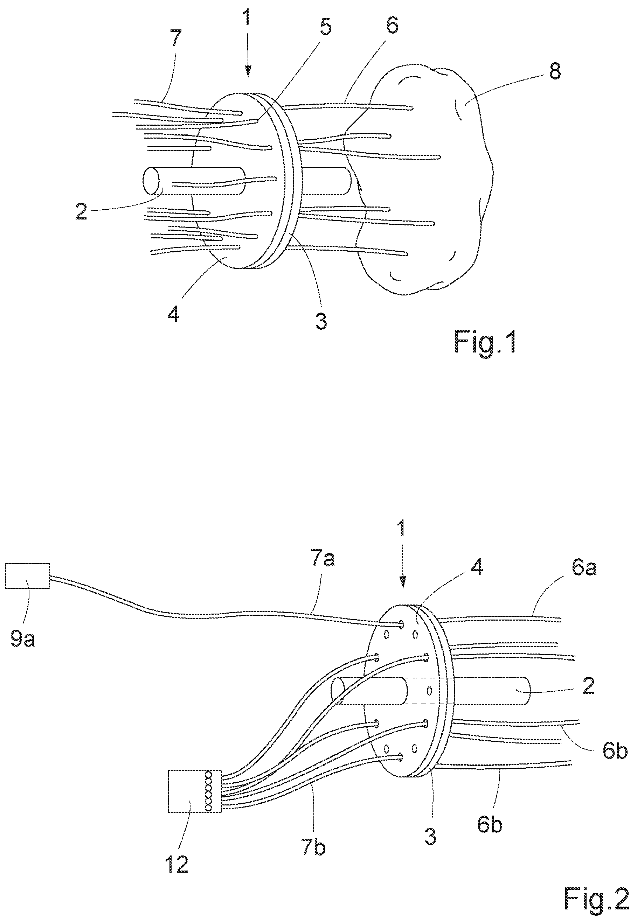

Fig 1 is a schematic view of a device for interactive interstitial photodynamic light therapy (PDT) or photo-thermal therapy (PTT) and photodynamic diagnosis (PDD) of a site on and/or in a human being or an animal. A plurality of, or at least two, radiation conductors, such as light guides or optical fibres 6 are directly inserted in a tissue 8, which may be an organ, a tumour or any other tissue. The fibres 6 may be inserted with their distal ends, respectively, in the tissue 8 in a geometric pattern and interstitially. Thus fibres 6 are patient fibres. As shown in Fig. 1, the distal ends of the patient fibres 6 are arranged in a geometric pattern covering a certain area to be investigated and/or treated. The proximal ends of the patient fibres 6 arranged at a distance from the tissue are inserted and attached to a flat disc 3 of a switching means 1, as shown in Fig 1. The flat disc 3 is arranged adjacent a second flat disc 4 and the flat discs are rotatable in relation to each other around a shaft 2.

A plurality of, or at least two, optical fibres 7 are connected to the second flat disc 4 in openings 5 as shown in Fig 1. A first diagnostic fibre 7a, see Fig. 2, is connected to a source of diagnostic light 9a at its proximal end arranged at a distance from plate 4. The diagnostic source may be a laser emitting light at a specific wavelength. Diagnostic light is passed from diagnostic light source 9a via diagnostic fibre 7a to patient fibre 6a via the two flat discs, suitably adjusted in relation to each other for good light transmission, and further to the tissue 8 via patient fibre 6a.

The diagnostic light is emitted through the distal end of patient fibre 6a into the tissue 8 and is scattered inside the tissue. The scattered diagnostic light is picked up by the distal ends of the remaining patient fibres 6b and passed therefrom to diagnostic sensors arranged in a diagnostic sensor unit 12. As is illustrated in Fig. 2, the diagnostic sensor unit 12 is connected to the second flat disc 4 via fibres 7b. The scattered light collected by the distal ends of patient fibres 6a is passed via patient fibres 6b and diagnostic fibres 7b, via the two flat discs 3, 4, respectively, to the diagnostic sensors in unit 12.

By rotating the second plate 4, the set of diagnostic fibres 7 are placed opposite the fibres 6 in a different order, whereupon another patient fibre 6 acts as transmitting fibre 6a and the remaining fibres act as receiving fibres, respectively.

The combined reply of the diagnostic sensors in unit 12 may be evaluated and a diagnostic image of the tissue 8 is thus obtained. Such diagnostic images may include information about the light flow through the tissue, autofluorescence of the tissue, or a fluorescence signal, which is obtained from a tumour sensitizer when the tissue is excited with visible or ultraviolet radiation. The last-mentioned fluorescence signal is shifted towards longer wavelength and clearly appears in contrast to the endogen fluorescence of the tissue. This information is used for localising tumours and for quantifying the size of the uptake of the sensitizer in the tissue. In this way, the correct light dose may be calculated or measured. Microthermistors may be arranged in connection with fibres 6 to measure temperature of the tissue, or temperature may be measured optically through specially prepared fibres.

The device in Figs. 1 and 2 may also be adjusted to a therapy position. In the therapy position, the proximal ends of all fibres may be connected to one or several laser sources, in order to obtain an efficient light radiation of the tissue to be treated. In this case therapeutic light sources may conveniently be fixed into the holes of flat disc 4, shown in Fig. 2, as illustrated in Fig. 1, and remote from disc 4 be connected to therapeutic light sources. Therapeutic light may thus be transmitted to the tissue 8 via a set of therapeutic fibres (a subset of fibres 7) to patient fibres 6 via the flat discs 4 and 3, respectively, and into tissue 8.

The light radiation may be infrared light, near-infrared light (NIR), visible light or ultraviolet (UV) light both in the therapy mode and the diagnostic mode.

The optical fibres, i.e. patient fibres 6, may be arranged directly into the tissue 8, or by means of insertion needles, usually in the form of a hollow cannula of metal with a sharp tip. Such a metal needle may be arranged in the correct position, for example by the aid of X-ray, ultrasound or visually.

An optical patient fibre 6 may be inserted distally through the lumen of the metal needle so that the tip of the optical fibre, at its distal end thereof, is arranged in the correct position towards tissue 8. The needle may be left in place or removed during PDT, PTT and PDD or interactive variants thereof. The optical fibre is normally covered by a lining or cladding, so that it is not exposed to body tissue and body fluids, and without leaking light radially, i.e. light is only transmitted or collected from the tip of the fibre. The proximal end at a distance from the tissue 8 of the optical patient fibre 6 is arranged in holes of the flat disc 3.

Since the needle for inserting the fibre is passed into the tissue 8, the needle may be used for adding an agent, which is usable for the subsequent diagnosis and/or therapy.

Another way to arrange the optical fibre in place may be to insert the needle as mentioned above, whereupon a plastic sleeve may be inserted over the needle, whereupon the needle is retracted and the optical fibre is subsequently inserted in the plastic sleeve. The optical fibre may then be permanently arranged in the sleeve. In this way, the plastic sleeve protects the optical fibre.

During medical treatment, patients often require medication, blood, or fluids. The most efficient way of administering these substances is by depositing them directly into the patient's blood stream where the circulatory system quickly directs the substance to the target tissue or organ. Administering a substance directly into a patient's blood stream is most commonly accomplished by injection with a conventional needle and syringe. During the course of treatment, however, a patient will often require repeated or continuous doses of medications. Repeated injections with conventional syringes damage blood vessels and cause significant discomfort to the patient.

Therefore, when a patient requires repeated doses of medication or other substances, catheters are commonly used in the health care profession. However, catheters are up to now not known for interstitial delivery of light to a tissue within systems for interactive interstitial photodynamic diagnosis and therapy of a tissue.

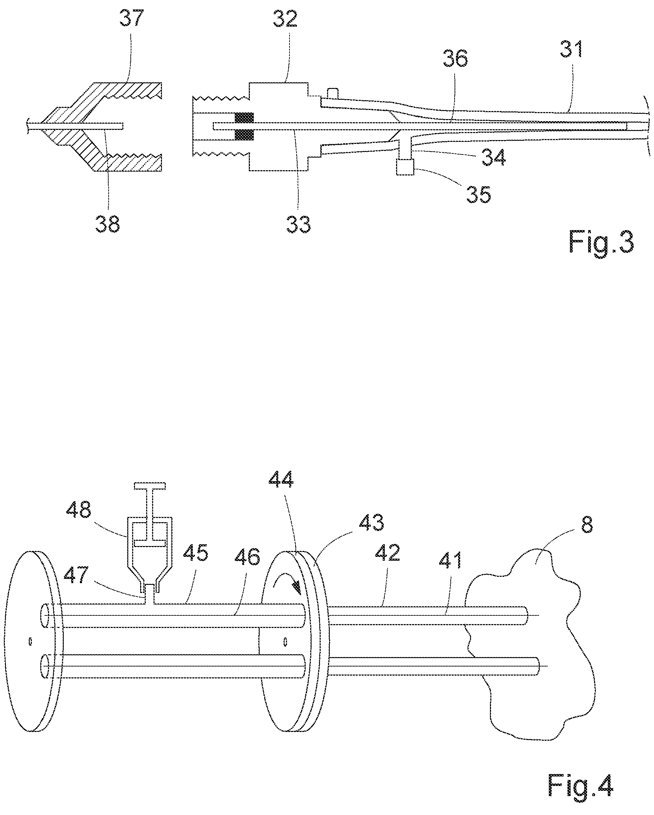

Hence, an alternative system to the system described above, is disclosed in Fig. 3, which comprises a sleeve of the Venflon® type intravenous access device; however, for a different use than conventionally known.

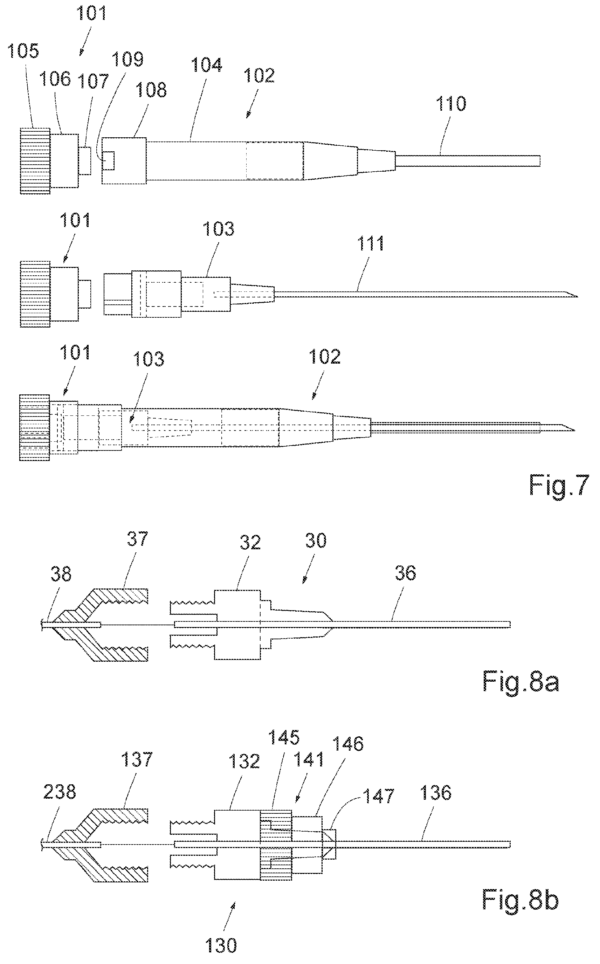

Generally, a Venflon® device, as shown in Fig. 7, may be classified as a ported intravenous (IV) catheter when it is in place in the patient. An IV catheter includes a catheter having a wall 110 defining a lumen, a proximal end, and a distal end, as well as a catheter adapter body that is in fluid communication with the catheter lumen. Initially a Venflon® device comprises furthermore an extremely thin needle 111 for inserting the catheter into a blood vessel. More precisely, "intravenous" means that a known Venflon® is configured for accessing the interior of a vein from outside the body, by means of the sharp needle that is put into a vein. Usually this is done for getting blood samples or for delivering drugs to the circulatory system via the veins. The needle is put into a vein close to the surface of the skin in either the arm or the back of a patients hand. This needle 111 is wrapped up with a soft plastic tube, arranged as a sleeve 110 around the needle. The needle makes the entry into the vein, and then is slid out leaving the soft plastic tube in place in communication with the vein. The drugs are put in through that tube 110, which then correctly is referred to as an "intraluminal indwelling catheter", but more commonly known by the tradename of Venflon®. The tube usually stays in place for a longer time, for instance throughout a surgical procedure.

However, it is pointed out that Venflon® devices are not supposed to be inserted interstitially into tissue. On the contrary, this has to be avoided, as for instance punctured veins leaking blood into the body are a serious threat to patient health. Catheters of this type, namely intravascular (IV) catheters, are conventionally used for infusing fluid, such as normal saline solution, various medicaments and total parenteral nutrition, into a patient, withdrawing blood from a patient or monitoring various parameters of the patient's vascular system, but not for inserting a light guide there through into a tissue. Peripheral IV catheters tend to be relatively short, and typically are on the order of about 5 cm or less in length.

In more detail, an IV catheter generally comprises a housing 102, an introducer needle 111, and a catheter 110, see Fig. 7. The housing is used to grip the vascular access device, e.g. at an gripping area 104, during catheter insertion. The introducer needle is attached to the proximal end of the catheter housing, e.g. by means of an intermediate catheter needle adaptor 103, and used to pierce the patient's skin and access the blood vessel. The catheter fits concentrically over the introducer needle and is held in place by friction engagement between the catheter needle adaptor 103 and the catheter housing 102. The relative lengths of the introducer needle and the catheter cannula are such that the tip of the introducer needle extends beyond the end of the catheter cannula when the introducer needle is attached to the housing. In use, a clinician pierces the patient's skin with the introducer needle and locates the patient's blood vessel. With the introducer needle in the patient's blood vessel, the clinician detaches the catheter needle adaptor 103 from the housing. The clinician then withdraws the introducer needle 111, leaving the catheter in place, and attaches an appropriate device to the catheter. The catheter is usually provided with a lock 101 to seal off the proximal end of the catheter device when needed during the procedure. Different lock mechanisms are available, usually in the form of a slide lock lid, e.g. a Luer lock mechanism. Lock 101 has a gripping area 105, an intermediate portion 106 and an insertion portion to fit into the proximal portion 108 of the catheter housing 102 or the catheter needle adaptor 103. Engagement of lock 101 with the catheter is provided by mating elements 109, 106 on the lock 101 and catheter housing 102 or catheter needle adaptor 103, respectively, e.g. a bayonet closing or mating threads.

According to an embodiment of the invention, a proximal end adapter for an IV catheter is provided. With reference to Figs. 3 and 8a, among others, the adapter has an adapter body 32, having proximal and distal ends, defining an axial lumen 33 extending between the proximal and distal ends. A catheter mounting element is provided at the distal end of the adapter body 32 for sealingly mounting a catheter to the body. An optical connector mounting element is provided at the proximal end of the adapter for mounting an optical connector 37 directly to the adapter body. Furthermore, the adapter has a light guide 36 that has proximal and distal ends. The light guide 36 is sealingly contained in said axial lumen defining an axial optical path extending between the proximal and distal ends of the adapter body 32.

In order to ensure that the light guide 38 transmitting light between the adapters 32 and 37, below also called main fibre, does not get contaminated during use, a further proximal, second adapter may be used to increase patient safety and ease of use. In this case, e.g. a SMA connector (SMA is an acronym for SubMiniature version A) (not shown in the Figs.) may be provided to connect proximally to fibre 38, which then distally is connected to fibre 36 by threading part 37 on part 32. Thus it is ensured that a fibre leading to an apparatus for providing or receiving light to or from fibre 36 is a true multiple use fibre. Fibre 36, and eventually the proximal thread of the adapter may be contaminated by body fluids during use. Hence the first adapter comprising parts 32, 36 may be a single use consumable device and be discarded after use. The first adapter may be made of a plastic material at low cost. The second adapter may be made have a metal housing and a glass light guide therein, and thus it may easily be sterilized. Hence the second adapter may be an adapter intended for multiple use. Instead of SMA type connectors also other commercially available optical connector types may be used, e.g. ST, FC or SC connector types. Optical connectors are used to mate a fiber to another fiber or to equipment.

Good coupling efficiency is to be provided by all connectors described herein, which requires precise positioning of the fibers in relation to each other. Connectors are used to provide a convenient system as the connection from an apparatus for photodynamic or photothermal therapy and/or diagnosis occasionally must be broken, for instance between patients or during therapy, e.g. to drain a tissue from fluid. The optical connectors described herein center fibers to each other so that their light transmitting part lies directly over the connector and in line.

According to an embodiment, this adapter is combined with a catheter that comprises a plastic and flexible sleeve 31 attached to the body portion 32 of the adapter. Instead of the body portion an insertion needle may be arranged in the catheter lumen. The needle is long enough to extend out of the plastic sleeve. The needle with the plastic sleeve is operated to pass into the skin and into the tissue. The plastic sleeve is of such a diameter that it fits tightly around the needle and is sufficiently rigid to follow the needle into the skin and tissue. When the plastic sleeve 31 has been placed in the desired position, the needle may be retracted leaving the catheter in place. Normally, the catheter is fixed to the skin by tape, so that it will not accidentally be removed. The catheter may comprise a port 34 that is normally closed by a lid 35. Agents or medicaments may be added to the plastic sleeve through said port, such as via a syringe. Such addition may be a bolus before the start of the diagnosis or therapy, intermittent addition or continuous addition of said agent. Moreover, excessive fluid may be drained from the tissue through the port. However, the port is optional according to some embodiments.

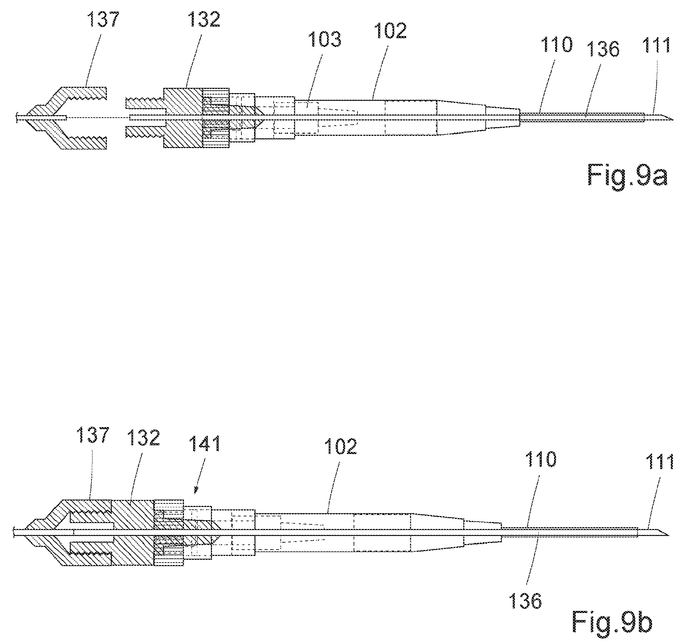

When the needle has been retracted, an optical fibre 36 may be inserted in place of the needle as shown in Fig. 3. Such insertion may take place after addition of a bolus, or take place before any addition of the agent. According to the present embodiment, the optical fibre attached to the adapter body 32, wherein this assembly is attached to the catheter. In this manner the optical fibre 36 is conveniently inserted into the catheter and towards the tissue to be diagnosed and treated. According to a specific embodiment, the distal end of the adapter body is attached to the proximal end of the catheter housing. The attachment may be done by means of mating Luer lock connectors, e.g. a male Luer connector on the proximal catheter housing and a female Luer connector on the distal adapter body 32, or vice versa. An example of an adapter having a Luer lock connection 141 is given in Fig. 8b, parts 145, 146, 147 correspond to parts 105, 106, 107, (Fig. 7) respectively. However, Luer lock coupling 141 is rotatably attached to adapter housing 132 for connecting to a catheter, as shown in Figs. 9a, 9b, 10a, 10b and 11-16 that are cross-sectional views of various proximal end adapter and catheter assemblies.

Finally, a connector 37 having an optical connector 38 may be attached to the proximal end of the adapter body 32, and thus to the catheter, in order to couple laser radiation or light into the optical fibre 36. The optical connector 37 has an optical fibre 38 centrally arranged, and it is at its other end connected to the flat disk 3, as shown in Fig. 1. Thus an optical path is ensured between the disk 3 and the tissue 8 via fibre 38, adapter 32 and fibre 36. The connection may conveniently be released and reattached by detaching adapter body 32 and thus fibre 36 from the catheter 31, e.g. for intermittent addition of new sensitizer to tissue 8. At the end of treatment, adapter 32 and catheter 31 may be discarded. Thus, contamination may be avoided by the adapter device when used for single treatment disposable use.

According to an embodiment, the catheter mounting element of the proximal end adapter at the distal end of the adapter body 32 comprises an internal tapered surface sized to matingly and sealingly engage an external tapered surface of a Luer lock connector.

According to another embodiment, the proximal end adapter has a radially compressible seal with a central bore receiving the light guide 36 therein. The seal is positioned within the adapter body along the axial lumen at a distance from the proximal end of the adapter, wherein the seal is radially outward compressed by said light guide to provide a sealing effect preventing leakage from the catheter through the adapter when mounted thereto.

According to an embodiment of the proximal end adapter, said light guide is sealingly attached in said axial lumen by a fastening means, such as glue, a clamp or local melting the adapter housing around the fibre 36.

According to an embodiment, a combination of the adapter a hollow catheter is provided. The catheter mounting element is adapted to connect the adapter body to the hollow catheter so the hollow catheter extends from the distal end of the adapter body. The proximal end of the light guide may be positioned within the adapter body at a distance from the proximal end of the adapter body, and the distal end of the light guide may extend from the distal end of the adapter body into the hollow catheter. The hollow catheter defines an axially extending catheter lumen, wherein the light guide may be radially in contact with the interior wall of the axially extending catheter lumen in order to provide a tight fitting therein. Alternatively, the light guide has a diameter less than the interior diameter of the catheter lumen, so that a coaxial, radially offset, channel is formed in the catheter lumen, when the light guide is inserted therein. The coaxial lumen may include an entrance port opening 34 into the portion of the axial lumen of the catheter body, e.g. for delivery of drugs.

The optical fibre 36 may end a few millimeters before the end of the sleeve 31, as shown in Fig. 3. In this way, light is emitted from the optical fibre and is spread by the sleeve before reaching the tissue.

In another embodiment, the optical fibre extends out of the end of the sleeve, whereby the optical fibre may be provided with a spreading device, such as a lens or a cone at the end of the optical fibre. Alternatively, the surface of the outer sector of the fibre may be ground to diffuse the light.

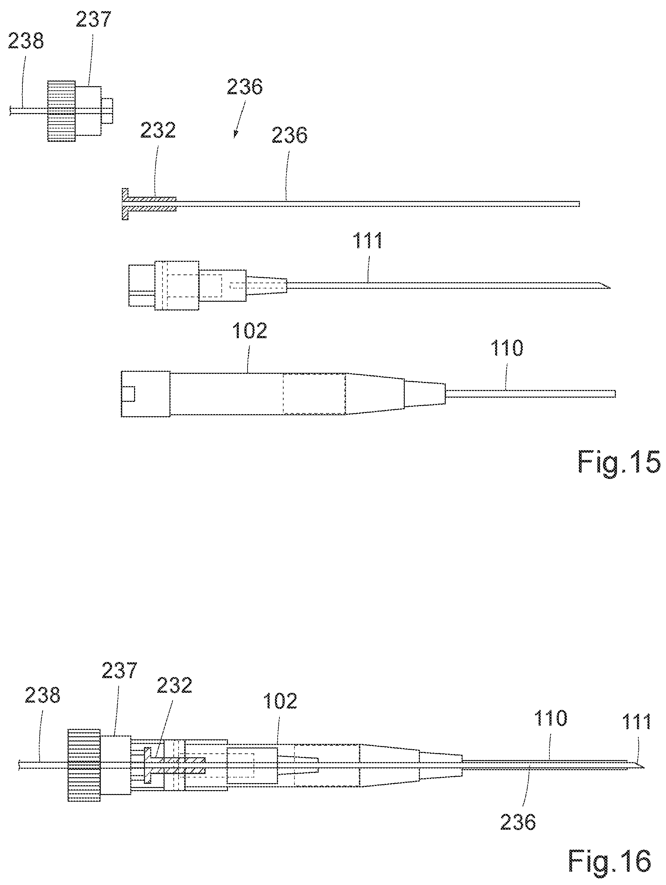

Fig. 9a shows the proximal end adapter 132 connected to a catheter body 102. In this embodiment the optical fibre 136 is arranged in the lumen 110 of catheter 102 and further inside the hollow needle 111. Fig. 9b illustrates how the optical connector 137 is threadably mounted on the proximal end of the adapter 132. In Fig. 9b and 16, the end of the optical fibre 136 or 236, respectively appears to converge into the tip of needle 111. However, in practice the needle is slightly wider than the optical fiber, in order to house the fibre therein; in contrast to the illustration of Fig. 9b and 16, where the tip of the needle due to the mentioned illustrative purposes appears to be part of the fibre 136 or 236, respectively.

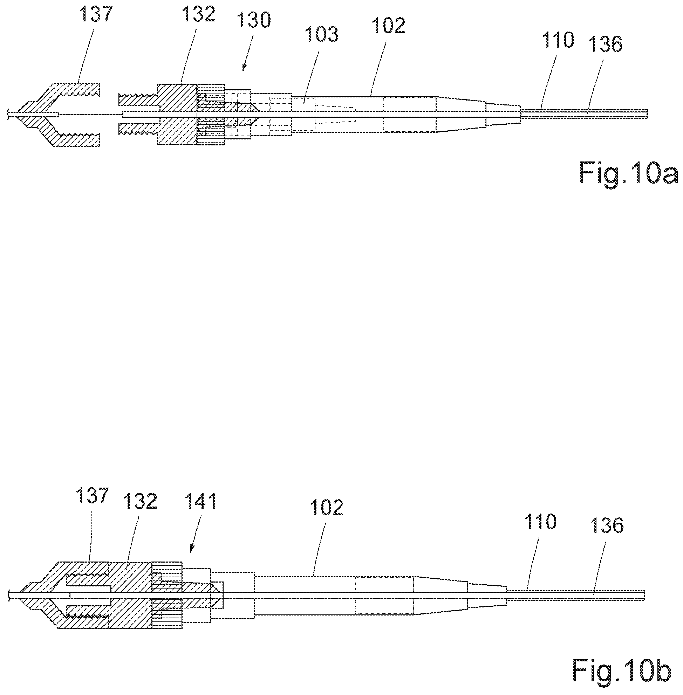

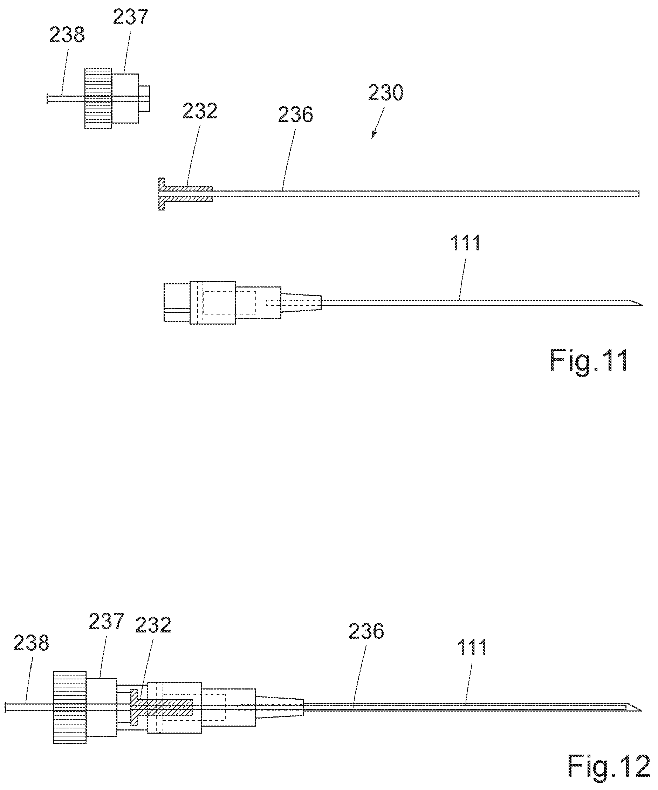

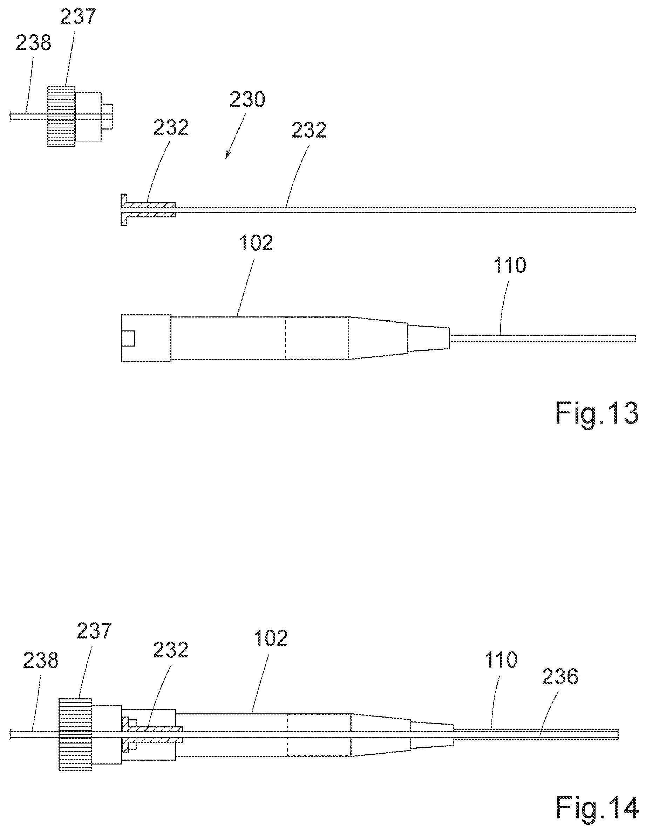

In Figs. 10a and 10b the fibre 136 is directly arranged in the lumen 110 of catheter 102, and the needle is omitted. Fig. 11 illustrates an end adapter 230 that is insertable into a hollow needle 111, see Fig. 12, or a catheter, see Figs. 13-16. Here, a Luer lock connector 237 comprises an optical fibre arranged therein for coupling to the fibre 236 of the adapter 230. Adapter 230 is inserted into needle 111 (Fig. 12) or lumen 110 (Fig. 14). Alternatively needle 111 with adapter 230 inside is arranged in catheter 102, see Fig. 16.

All embodiments of the adapter described herein may advantageously be used in systems as described above with reference to Figs. 1 and 2.

Fig. 4 discloses a system in addition adding a substance to a tissue at photodynamic diagnosis or therapy or photo-thermal therapy. Several optical fibres 41 have been arranged with a distal tip portion ending in a tissue 8 to be treated and extending through sleeves 42, which may be flexible or more or less rigid. The end of each optical fibre being distal from the tissue 8 and the corresponding end of the sleeve is attached to a plate 43 which may be similar to plate 3 shown in Fig. 1. While only two have been shown in Fig. 4, any number of fibres may be used. The sleeve may be of a plastic material or a metal.

Another plate 44 is arranged adjacent to plate 43. Plate 44 may be similar to plate 4 in Fig. 1. A sleeve 45 including an optical fibre 46 is connected to plate 44. Optical fibre 46 may be similar to optical fibre 7a shown in Fig. 2.

Sleeve 45 is provided with a port 47 which may be normally closed by a lid (not shown) and a syringe 48 may be attached to the port. The syringe may deliver a substance or an agent to the tissue 8 through the port 47 and the sleeves 45 and 42. The supply may be a bolus, or intermittent or continuous supply. The supply may take place before or during the diagnosis or during the therapy.

In Fig. 4, only one sleeve is shown provided with a port 47. Such sleeve may be connected to each sleeve 42 during the diagnosis step as described above. Alternatively, several or all sleeves 45 are provided with ports.

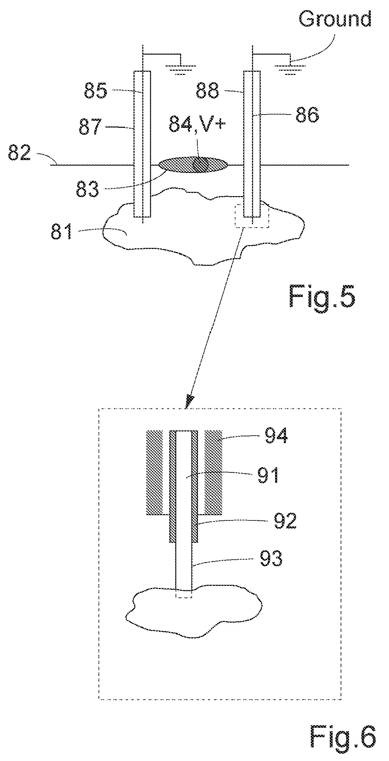

Fig. 5 discloses a further method for supplying a substance to the tissue. The optical fibre may be covered by a lining having the property to conduct electric current. This property may be used for electrophoretic supply of a substance to the tissue. The ends of the optical fibres are arranged in the tissue as described above with reference to Fig. 3.

The optical fibre is in an embodiment covered by a lining of a suitable metal, such as silver (Ag). The metal lining serves multiple purposes.
1) The metal lining operates as a total reflection medium, which means that the optical fibre may be arranged directly in the tissue, without a protecting sleeve. The optical fibre may be arranged in direct contact with any fluid present around the tissue.
2) The metal is a conductor for electricity, which means that the optical fibre can be used as an electrode, for example for iontophoretic introduction of a substance in the tissue.

Fig. 5 is a schematic diagram of a system for introduction of an agent to a tissue by iontophoresis. A tumour 81 has been identified below the skin 82 of a patient. The tumour may be positioned close to the skin or at a substantial depth. The effect of iontophoretic introduction of a substance may be larger if the tumour to be treated is located close to the skin.

A pad 83 comprising the agent to be introduced is arranged on the skin surface above the tumour to be treated. The pad may at the same time act as an electrode as indicated by a connector 84 to a positive contact of an electric current.

Two optical fibres 85, 86 covered with a metal layer are shown in Fig. 5 and are arranged in plastic sleeves 87, 88. However, any number of optical fibres and sleeves may be used, such as three, four, five or six fibres and sleeves. The fibres 86, 87 may be arranged in the tissue 81 by means of the above described adapter 32 and catheter 31. Each sleeve is at one end connected to a plate, similar to the plate 43 in Fig. 4, and at the other end terminated at a position in the tissue of tumour 81. The optical fibres 85,86 extend through the sleeves 87,88. At the end distal from the tumour, each optical fibre is electrically connected to a ground potential. Thus, the distal end extends out of the sleeve into contact with the tissue. An electric current is generated between the pad 83 and each end of the fibres. The current carries the agent through the skin and into the tumour. The agent may be ALA-solution, phtalocyanines, chlorines etc. For certain agents the polarity may be reversed.

These agents act in the following manner for therapy of the tumour.

ALA or aminolevulinic acid is a precursor photosensitizer. When applied, ALA is gathered in the tumour cells, due to their high activity, and is converted into protoporphyrin IX (PPIX), which is a photosensitizer. By applying a laser beam, at a wavelength of approximately 635nm, on the tumour, the PPIX is excited contributing to excite the oxygen, in the cells, to its singlet state. The singlet state oxygen will fatally damage the cells and within a few days the cells in the affected area are dead.

Phtalocynanines are a second generation of photosensitizers with improved pharmaceutical profiles. They have a strong absorption in the red region in which tissue is rather transparent making them suitable for PDT.

Chlorines are also a second generation photosensitizer suitable for PDT.

Other sensitizers are Foscan and Tookad.

The metal layers, notably silver, in contact with the tissue or tumour may act as a bacteriostatic matter preventing infections.

As shown in Figs. 5 and 6, the distal end of the fibre extends out of the plastic sleeve into contact with the tissue. This distal end 93 may also be free from the lining of metal, at least over the end portion thereof. Fig. 6 shows an optical fibre 91 covered by a metal lining 92, which is able to reflect the light passing inside the fibre and act as an electric current conducting layer. The fibre is arranged inside a plastic sleeve 94, which protects the fibre and forms an insulation in relation to the body portion surrounding the sleeve. Because the end 93 is not covered by silver coating and is in contact with a fluid in the tissue, the light in the fibre will partially pass out through the side walls of the fibre. Thus, the end 93 will diffuse the light. This is an advantage in PDT and PDD.

When the optical fibre is used for optical diagnosis or therapy, the light passing through the fibre is spread in the area surrounding the end or collected therefrom, which is an advantage. The same is true at light therapy.

The methods of supplying the agent as described above may be used as alternatives or in combination.

The sleeves 87 and 88 may be a plastic sleeve or a tube of a material that is compatible with the human being. It may be more or less flexible or elastic.

The sleeve 87,88 may be a needle of steel attached to a plate, similar to the plate 43 in Fig. 4, in a suitable configuration. There may be 6 needles arranged in a geometric configuration. The needles are pushed through the skin and into the tumour.

The sleeves may be made from polyurethane or polyvinyl chloride with a suitable softener. The sleeves may incorporate barium, so that the positioning of the sleeves may be monitored by X-ray.

In a further embodiment, each sleeve is a so called Venflon® device, which is of the intravenous catheter type described above.

In cases, where no administration of agent needs to take place through a sleeve, the sleeve may be dispensed with and the fibre may pass directly to the tissue. In this case it may be advantageous that the metal lining is covered by an electrically insulating layer leaving only a small exposed area of the silver lining at the end, so that iontophoretic administration of substance may be possible.

As used herein, the term "proximal" refers to a location on the catheter that, during normal use, is closest to the clinician using the device and farthest from the patient in connection with whom the device is used. Conversely, the term "distal" refers to a location on the catheter that, during normal use, is farthest from the clinician using the device and closest to the patient in connection with whom the device is used.

Reference has been made to threads and tapered surfaces suitable for use with Luer lock type connectors. Generally, Luer locks have a 6% included angle taper as fully characterized in publication ISO 594/1, First Edition, 1986-06-15 and made part of this application by reference. Luer lock connectors have a double start, righthand thread described in publication ISO 594-2, First Edition, 1991-05-01 made part of this application by reference.

Herein above, a specific embodiment of the invention has been described with reference to the drawings. However, the invention can be varied within the embodiments shown. The different separate features may be combined in other combinations than specifically disclosed. The invention is only limited by the appended patent claims.

### Preferred embodiment

1. A proximal end adapter for a catheter comprising
   an adapter body (32), having proximal and distal ends, defining an axial lumen extending between the proximal and distal ends, **characterized by**
   a catheter mounting element at the distal end of the adapter body (32) for mounting, such as sealingly mounting, a catheter to the body;
   an optical connector mounting element at the proximal end for mounting an optical connector (37) to the adapter body (32); and
   a light guide (36) having proximal and distal ends, wherein said light guide is sealingly contained in said axial lumen defining an axial optical path extending between the proximal and distal ends of the adapter body,
   and wherein said proximal end adapter is configured for use in a system for interactive interstitial photodynamic or photothermal therapy and/or diagnosis of a tissue (8).
2. The proximal end adapter according to claim 1, wherein said optical connector mounting element at the proximal end for mounting an optical connector (37) to the adapter body is configured to directly mount to said adapter body.
3. The proximal end adapter according to claim 1 or 2, wherein said catheter mounting element at the distal end of the adapter body (32) comprises a mounting surface to sealingly engage said optical connector (37).
4. The proximal end adapter according to claim 3, wherein said mounting surface is an internal tapered surface sized to matingly and sealingly engage an external tapered surface of a Luer lock connector comprised in said optical connector.
5. The proximal end adapter according to claim 3, wherein said mounting surface is threaded for matingly and sealingly engage a threaded surface comprised in said optical connector.
6. The proximal end adapter according to any of claims 1 to 5, comprising a radially compressible seal having a central bore receiving the light guide therein, positioned within the adapter body along the axial lumen at a distance from the proximal end, wherein the seal is radially outward compressed by said light guide to provide said sealing.
7. The proximal end adapter according to any of claims 1 to 5, wherein said light guide is sealingly attached in said axial lumen by a fastening means, such as glue.
8. The proximal end adapter according to any of the preceding claims, wherein said catheter is a transcutaneous intravenous catheter device.
9. The proximal end adapter according to claim 8, wherein said catheter has a length of approximately less than 10 cm.
10. The proximal end adapter according to any of the preceding claims, wherein said adapter when mounted to said catheter seals said lumen against fluid connection from said proximal side of said adapter and provides light connection from said proximal side of said adapter to said lumen via said light guide.
11. A combination of the adapter of any of claims 1 to 10 and a hollow catheter, wherein the catheter mounting element is adapted to connect the adapter body (32) to the hollow catheter so that the hollow catheter extends from the distal end of the adapter body.
12. The combination of claim 11, wherein the proximal end of the light guide is positioned within the adapter body at a distance from the proximal end of the adapter body, and wherein the distal end of the light guide extends from the distal end of said adapter body into the hollow catheter.
13. The combination of claim 12, wherein the hollow catheter defines an axially extending catheter lumen, wherein the light guide is radially in contact with the interior wall of the axially extending catheter lumen.
14. The combination of claim 12, wherein the hollow catheter defines an axially extending catheter lumen, wherein the light guide has a diameter less than the interior diameter of the catheter lumen, so that a coaxial, radially offset, channel is formed in the catheter lumen.
15. The combination of claim 14, wherein the secondary lumen includes an entrance port opening into a portion of the axial lumen of the catheter body.
16. The combination of claim 11, further comprising a needle that is inserteable and retractable in the hollow catheter for arranging said catheter to said tissue when said adapter is disconnected from said catheter, such that said radiation fibre is inserteable through the catheter or the needle towards said tissue (8).
17. A system configured for photodynamic or photothermal therapy and/or photodynamic diagnosis of a tissue (8), said system in combination comprising an assembly of:
   a proximal end adapter for a catheter comprising
   an adapter body (32), having proximal and distal ends, defining an axial lumen extending between the proximal and distal ends,
   a catheter mounting element at the distal end of the adapter body (32) for sealingly mounting a catheter to the adapter body,
   and a hollow catheter (31), wherein the catheter mounting element connects a catheter body to the hollow catheter so that the hollow catheter extends from the distal end of the catheter body;
   a first light guide (36) having proximal and distal ends, wherein said first light guide is sealingly contained in said axial lumen defining an axial optical path extending between the proximal and distal ends of the adapter body (32) towards said tissue (8) and into close proximity thereof or interstitially into said tissue;
   an optical connector mounting element at the proximal end having mounted an optical connector (37) to the adapter body, and
   wherein said optical connector (37) is coupled to a light source via a second light guide (38),
   such that light from said light source is transmittable from said light source via said second light guide (38) and further via said optical connector (37) to said first light guide (36) via said proximal end adapter towards said tissue (8) through said catheter (31) for said therapy and/or diagnosis of said tissue (8).
18. The system according to claim 17, wherein said first light guide (36) is inserteable into said catheter (31) after arranging said sleeve in said tissue.
19. The system according to claim 17, further comprising a supply device for supplying an agent via said catheter (31) to said tissue (8).
20. The system according to claim 19, wherein said catheter (31) is provided with a port (34) for supply of said agent.
21. The system according to claim 17, further comprising:
   a pad including said agent and arranged at the skin adjacent said tissue and forming a first electrode connected to a first electric potential, whereby a second electric potential is connected to an electrically conductive lining arranged on at least one of said radiation fibres, to form a current for introduction of said agent into said tissue by iontophoresis.
22. The system according to claim 21, wherein said conductive lining is of a metal, such as silver.
23. The system according to any of claims 17 to 22, comprising a second adapter for releasably connecting the proximal end of said adapter body to a further optical connector proximally arranged at said optical connector (37).
24. A method for use of a proximal end adapter of a catheter, the adapter having an adapter body (32), having proximal and distal ends, defining an axial lumen extending between the proximal and distal ends, and a catheter mounting element at the distal end of the adapter body (32) for mounting a catheter to the body; an optical connector mounting element at the proximal end for mounting an optical connector (37) to the adapter body; and a light guide having proximal and distal ends, wherein said light guide is sealingly contained in said axial lumen defining an axial optical path extending between the proximal and distal ends of the adapter body, said method comprising:
   mounting the adapter to a catheter housing by said catheter mounting element, to provide the light guide in a lumen of the catheter;
   mounting an optical connector to said optical connector mounting element of said adapter, thus
   providing an optical path via said adapter.
25. The method according to claim 24, wherein said mounting the adapter and said mounting of the optical connector comprises threadably mounting.
26. A method for photodynamic or photothermal therapy and/or photodynamic diagnosis of a tissue, comprising
   providing at least one source of laser radiation, and
   providing at least one radiation fibre (36) for transmitting the laser radiation to said tissue
   **characterized by**
   arranging a catheter interstitially in said tissue;
   mounting a proximal end adapter to the proximal end of the catheter, said adapter having a radiation fibre thus mounted in a lumen of the catheter to said tissue,
   connecting said adapter to a distal radiation fibre (38) via an optical connector (37) coupled to said at least one source of laser radiation,
   such that when said adapter is connected to said connector (37) laser radiation from said at least one source of laser radiation is transmitted via said distal optical fibre (38) to said at least one radiation fibre (36) and said tissue.
27. The method according to claim 26, prior to mounting the adapter to the catheter comprises
   inserting a needle into the tissue, said needle having a catheter sleeve arranged over said needle,
   removing said needle, and inserting at least one radiation fibre in said sleeve.
28. The method according to claim 26 or 27, comprising
   retracting of said a proximal end adapter from said lumen, and
   draining liquid through said lumen, and re-mounting the proximal end adapter to the proximal end of the catheter.
29. Use of an intravenous access device for interstitially coupling light to a tissue.
30. Use according to claim 29 comprising photodynamic or photothermal therapy and/or photodynamic diagnosis of said tissue.

## Claims

1. A system configured for photodynamic or photothermal therapy and/or photodynamic diagnosis of a tissue (8), said system in combination comprising an assembly of:
a hollow catheter (31) and a proximal end adapter for said hollow catheter;
said proximal end adaptor comprising
an adapter body (32), having proximal and distal ends, defining an axial lumen extending between the proximal and distal ends, and
a catheter mounting element at the distal end of the adapter body (32) where said adapter body is releasably mountable to said hollow catheter, whereby said catheter mounting element is releasably connectable to said hollow catheter and said hollow catheter extends from the distal end of the adaptor body when mounted thereto;
said assembly further comprising:
a first light guide (36) having a proximal end and a distal end, wherein said first light guide is sealingly attached to said proximal end adapter in said axial lumen and defines an axial optical path extending between the proximal and distal ends of the adapter body (32) towards said tissue (8) and into close proximity thereof or interstitially into said tissue, wherein the proximal end of said first light guide (36) is configured to be positioned within the adapter body (32), and said distal end of said first light guide extends out of said distal end of said adapter body and into said catheter,
an optical connector (37) and an optical connector mounting element for mounting said optical connector (32) at the proximal end of said adaptor body (32), and
wherein said optical connector (37) comprises a second light guide (38), and is coupled to a light source via said second light guide (38),
such that light from said light source is transmittable from said light source via said second light guide (38) to the distal end thereof and further via said optical connector (37) to the proximal end of said first light guide (36) and via said proximal end adapter towards said tissue (8) through said hollow catheter (31) for said therapy and/or diagnosis of said tissue (8).

2. The system according to claim 1, wherein said first light guide (36) is inserteable into said hollow catheter (31) after arranging a sleeve of said hollow catheter in said tissue.

3. The system according to any of claims 1 or 2, wherein said second light guide (38) is a multiple use fibre.

4. The system according to any of claims 1 to 3, further comprising a supply device for supplying an agent via said hollow catheter (31) to said tissue (8).

5. The system according to any of claims 1 to 4, further comprising a supply device for supplying an agent via said hollow catheter (31) to said tissue (8) and, wherein said hollow catheter (31) is provided with a port (34) for supply of said agent.

6. The system according to any of claims 1 to 5, comprising a second adapter having a further optical connector provided to connect proximally to said second light guide (38) for releasably connecting the proximal end of said adapter body to said further optical connector proximally arranged at said optical connector (37).

7. The system according to any of claims 1 to 6, wherein said optical connector mounting element at the proximal end is configured for directly mounting said optical connector (37) to the adapter body.

8. The system according to any of claims 1 to 7, wherein said catheter mounting element at the distal end of the adapter body (32) comprises a mounting surface for sealingly engaging said catheter.

9. The system according to claim 8, wherein said mounting surface is an internal tapered surface sized to matingly engage and seal an external tapered surface of a Luer lock connector comprised in said catheter; or wherein said mounting surface is threaded for matingly engage and seal a threaded surface comprised in said catheter.

10. The system according to any of claims 1 to 9, wherein said seal means is a radially compressible seal having a central bore receiving the light guide therein, positioned within the adapter body along the axial lumen at a distance from the proximal end, wherein the seal is radially outward compressible by said light guide to provide said sealing; or wherein said seal means comprises a fastening means such that said light guide is sealingly attached in said axial lumen by said fastening means, such as glue; and/or wherein said catheter mounting element is adapted to mount a transcutaneous intravenous catheter device; or wherein said catheter mounting element is adapted to mount a transcutaneous intravenous catheter device, and wherein said catheter has a length of approximately less than 10 cm; and/or wherein said adapter when mounted to said catheter seals said lumen against fluid connection from said proximal side of said adapter and provides light connection from said proximal end of said adapter to said lumen via said light guide.

11. The system according to any of claims 1 to 10, further comprising a needle that is inserteable and retractable in the hollow catheter for arranging said catheter to said tissue when said adapter is disconnected from said catheter, such that said light guide is inserteable through the catheter or the needle towards said tissue (8).

12. The system according to any of claims 1 to 11, further comprising that said distal end of said first light guide is radially in contact with the interior wall of an axially extending lumen of said catheter.

13. A method for mounting a proximal end adapter of a catheter, the proximal end adapter having an adapter body (32), having proximal and distal ends, defining an axial lumen extending between the proximal and distal ends defining an axial optical path extending between the proximal end distal ends of the adapter body, and a catheter mounting element at the distal end of the adapter body (32) for mounting a catheter to the adaptor body; an optical connector mounting element at the proximal end for mounting an optical connector (37) to the adapter body; and a first light guide (36) having proximal and distal ends, wherein said first light guide (36) is sealingly contained by sealing means in said axial lumen (33), said method comprising the following steps:
positioning said first light guide (36) with the proximal end thereof within the adapter body (32),
mounting the proximal end adapter to a catheter housing by said catheter mounting element, to provide said first light guide in a lumen of the catheter;
mounting an optical connector (37) to said optical connector mounting element of said adapter body (32), thereby coupling a distal end of a second optical light guide (38) comprised in said optical connector (37) to the proximal end of said first light guide (36), thus
providing an optical path via said adapter.

## Patentansprüche

1. System, das für die photodynamische oder photothermische Therapie und/oder die photodynamische Diagnostik von Gewebe (8) eingerichtet ist, wobei das System in Kombination eine Anordnung von folgenden [Teilen] umfasst:
einen Hohlkatheter (31) und einen proximalen Endadapter für den Hohlkatheter;
wobei der proximale Endadapter einen Adapterkörper (32) umfasst, der ein proximales und ein distales Ende aufweist, wodurch ein axiales Lumen definiert wird, welches sich zwischen dem proximalen und dem distalen Ende erstreckt, und
ein Katheter-Befestigungselement an dem distalen Ende des Adapterkörpers (32), an dem sich der Adapterkörper auf freigebbare Weise an dem Hohlkatheter befestigen lässt, wodurch sich das Katheter-Befestigungselement auf freigebbare Weise an dem Hohlkatheter befestigen lässt und sich der Hohlkatheter von dem distalen Ende des Adapterkörpers erstreckt, wenn er an diesen angebracht ist;
wobei die Anordnung ferner umfasst;
einen ersten Lichtleiter (36), der ein proximales Ende und ein distales Ende aufweist, wobei der erste Lichtleiter innerhalb des axialen Lumens auf abdichtende Weise an dem proximalen Endadapter befestigt ist und einen axialen optischen Pfad definiert, welcher sich zwischen dem proximalen und dem distalen Ende des Adapterkörpers (32) zu dem Gewebe (8) hin und in die unmittelbare Nähe von diesem oder interstitiell in das Gewebe hinein erstreckt, wobei das proximale Ende des ersten Lichtleiters (36) so eingerichtet ist, dass es innerhalb des Adapterkörpers (32) positioniert werden kann, und sich das distale Ende des ersten Lichtleiters aus dem distalen Ende des Adapterkörpers und in den Katheter hinein erstreckt,
einen optischen Stecker (37) und ein Befestigungselement für den optischen Stecker zum Befestigen des optischen Steckers (32) an dem proximalen Ende des Adapterkörpers (32), und
wobei der optische Stecker (37) einen zweiten Lichtleiter (38) umfasst, und über den zweiten Lichtleiter (38) an eine Lichtquelle gekoppelt ist,
so dass das Licht von der Lichtquelle sich zum Zwecke der Therapie und/oder der Diagnostik des Gewebes (8) von der Lichtquelle über den zweiten Lichtleiter (38) zu dessen distalem Ende und weiter über den optischen Stecker (37) zu dem proximalen Ende des ersten Lichtleiters (36) und über den proximalen Endadapter durch den Hohlkatheter (31) zu dem Gewebe (8) hin übertragen lässt.

2. System gemäß Anspruch 1, wobei sich der erste Lichtleiter (36) in den Hohlkatheter (31) einfügen lässt, nachdem eine Hülse des Hohlkatheters in dem Gewebe angeordnet worden ist.

3. System gemäß einem beliebigen der Ansprüche 1 oder 2, wobei es sich bei dem zweiten Lichtleiter (38) um eine Mehrfachnutzungs-Faser handelt.

4. System gemäß einem beliebigen der Ansprüche 1 bis 3, ferner eine Zufuhrvorrichtung zur Zufuhr eines Wirkstoffes über den Hohlkatheter (31) zu dem Gewebe (8) hin umfassend.

5. System gemäß einem beliebigen der Ansprüche 1 bis 4, ferner eine Zufuhrvorrichtung zur Zufuhr eines Wirkstoffes über den Hohlkatheter (31) zu dem Gewebe (8) hin umfassend, und wobei der Hohlkatheter (31) zur Zufuhr des Wirkstoffes mit einem Port (34) ausgestattet ist.

6. System gemäß gemäß einem beliebigen der Ansprüche 1 bis 5, einen zweiten Adapter umfassend, der einen weiteren optischen Stecker aufweist, der vorgesehen ist, um proximal an den zweiten Lichtleiter (38) angeschlossen zu werden, so dass das proximale Ende des Adapterkörpers auf freigebbare Weise an den weiteren optischen Stecker angeschlossen werden kann, der proximal an dem optischen Stecker (37) angeordnet ist.

7. System gemäß einem beliebigen der Ansprüche 1 bis 6, wobei das Befestigungselement für den optischen Stecker an dem proximalen Ende so eingerichtet ist, dass es den optischen Stecker (37) direkt an dem Adapterkörper befestigt.

8. System gemäß einem beliebigen der Ansprüche 1 bis 7, wobei das Katheter-Befestigungselement an dem distalen Ende des Adapterkörpers (32) eine Befestigungsfläche umfasst, die dazu dient, mit dem Katheter auf abdichtende Weise einzurasten.

9. System gemäß Anspruch 8, wobei es sich bei der Befestigungsfläche um eine sich verjüngende innere Fläche handelt, die eine derartige Größe aufweist, dass sie auf eine ineinandergesteckte Weise mit einer sich verjüngenden externen Oberfläche eines Luer-Lock-Steckers, welcher in dem Katheter enthalten ist, einrastet und diese abdichtet; oder wobei die Befestigungsfläche mit einem Gewinde verstehen ist, um mit einer mit einem Gewinde versehenen Oberfläche, welche in dem Katheter enthalten ist, einzurasten und diese abzudichten.

10. System gemäß einem beliebigen der Ansprüche 1 bis 9, wobei es sich bei dem Dichtungsmittel um eine radial komprimierbare Dichtung handelt, die eine zentrale Bohrung aufweist, in welcher der Lichtleiter aufgenommen wird, die innerhalb des Adapterkörpers entlang des axialen Lumens in einem Abstand zu dem proximalen Ende positioniert ist, wobei sich die Dichtung durch den Lichtleiter radial nach außen komprimieren lässt, um die Abdichtung zu gewährleisten; oder wobei das Dichtungsmittel ein Befestigungsmittel umfasst, so dass der Lichtleiter durch das Befestigungsmittel, wie beispielsweise Klebstoff, auf abdichtende Weise in dem axialen Lumen befestigt ist; und/oder wobei das Katheter-Befestigungselement zum Befestigen einer transkutanen intravenösen Kathetervorrichtung angepasst ist;
oder wobei das Katheter-Befestigungselement zur Befestigung einer transkutanen intravenösen Kathetervorrichtung angepasst ist, und wobei der Katheter eine Länge von ungefähr weniger als 10 cm aufweist; und/oder wobei der Adapter, sofern er an dem Katheter befestigt ist, das Lumen gegen eine Fluidverbindung von der proximalen Seite des Adapters abdichtet und über den Lichtleiter eine Lichtverbindung von dem proximalen Ende des Adapters zu dem Lumen bereitstellt.

11. System gemäß einem beliebigen der Ansprüche 1 bis 10, ferner eine Nadel umfassend, die sich in den Hohlkatheter einführen und wieder aus diesem herausziehen lässt, um den Katheter an dem Gewebe anzuordnen, wenn der Adapter von dem Katheter abgetrennt wird, so dass sich der Lichtleiter durch den Katheter oder die Nadel zu dem Gewebe (8) hin einführen lässt.

12. System gemäß einem beliebigen der Ansprüche 1 bis 11, ferner umfassend, dass das distale Ende des ersten Lichtleiters radial in Kontakt mit der Innenwand des sich axial erstreckenden Lumens des Katheters steht.

13. Verfahren zur Befestigen eines proximalen Endadapters eines Katheters, wobei der proximale Endadapter einen Katheterkörper (32) aufweist, welcher ein proximales und ein distales Ende hat, wodurch ein axiales Lumen definiert wird, das sich zwischen dem proximalen und dem distalen Ende erstreckt, wodurch ein axialer Strahlengang definiert wird, der sich zwischen dem proximalen [und] dem distalen Ende des Adapterkörpers erstreckt, und ein Katheter-Befestigungselement an dem distalen Ende des Adapterkörpers (32) zur Befestigung eines Katheters an dem Adapterkörper; ein Befestigungselement für einen optischen Stecker an dem proximalen Ende zur Befestigung eines optischen Steckers (37) an dem Adapterkörper; und einen ersten Lichtleiter (36), der ein proximales und ein distales Ende aufweist, wobei der erste Lichtleiter (36) durch Dichtungsmittel auf abdichtende Weise innerhalb des axialen Lumens (33) gehalten wird, wobei das Verfahren die folgenden Schritte umfasst:
Positionierung des ersten Lichtleiters (36) mit dessen proximalem Ende innerhalb des Adapterkörpers (32),
Befestigen des proximalen Endadapters an einem Kathetergehäuse durch das Katheter-Befestigungselement, so dass ein erster Lichtleiter in einem Lumen des Katheters vorgesehen wird;
Befestigen eines optischen Steckers (37) an dem Befestigungselement für den optischen Stecker des Adapterkörpers (32), wodurch ein distales Ende eines zweiten optischen Lichtleiters (38), welcher in dem optischen Stecker (37) enthalten ist, an dem proximalen Ende des ersten Lichtleiters (36) gekoppelt wird, wodurch die Bereitstellung eines Strahlengangs über den Adapter erfolgt.

## Revendications

1. Système configuré pour une thérapie photodynamique ou photothermique et/ou un diagnostic photodynamique d'un tissu (8), ledit système en combinaison comprenant un assemblage de :
un cathéter creux (31) et d'un adaptateur d'extrémité proximale pour ledit cathéter creux,
ledit adaptateur d'extrémité proximale comprenant
un corps d'adaptateur (32), ayant des extrémités proximale et distale, définissant une lumière axiale s'étendant entre les extrémités proximale et distale, et
un élément de montage de cathéter au niveau de l'extrémité distale du corps d'adaptateur (32) où ledit corps d'adaptateur peut être monté avec possibilité de libération sur ledit cathéter creux, grâce à quoi ledit élément de montage de cathéter peut être connecté avec possibilité de libération sur ledit cathéter creux et ledit cathéter creux s'étend à partir de l'extrémité distale du corps d'adaptateur lorsqu'il est monté sur celui-ci,
ledit assemblage comprenant en outre :
un premier conduit de lumière (36) ayant une extrémité proximale et une extrémité distale, dans lequel ledit premier conduit de lumière est fixé de façon étanche audit adaptateur d'extrémité proximale dans ladite lumière axiale et définit un trajet optique axial s'étendant entre les extrémités proximale et distale du corps d'adaptateur (32) en direction dudit tissu (8) et à proximité étroite de celui-ci ou de manière interstitielle dans ledit tissu, dans lequel l'extrémité proximale dudit premier conduit de lumière (36) est configurée pour être positionnée dans le corps d'adaptateur (32), et ladite extrémité distale dudit premier conduit de lumière s'étend hors de ladite extrémité distale dudit corps d'adaptateur et dans ledit cathéter,
un connecteur optique (37) et un élément de montage de connecteur optique destiné à monter ledit connecteur optique (32) au niveau de l'extrémité proximale dudit corps d'adaptateur (32),
dans lequel ledit connecteur optique (37) comprend un deuxième conduit de lumière (38) et est couplé à une source de lumière par le biais dudit deuxième conduit de lumière (38),
de sorte que de la lumière provenant de ladite source de lumière puisse être transmise de ladite source de lumière par le biais dudit deuxième conduit de lumière (38) vers l'extrémité distale de celui-ci et en outre par le biais dudit connecteur optique (37) vers l'extrémité proximale dudit premier conduit de lumière (36) et par le biais dudit adaptateur d'extrémité proximale en direction dudit tissu (8) à travers ledit cathéter creux (31) pour ladite thérapie et/ou ledit diagnostic dudit tissu (8).

2. Système selon la revendication 1, dans lequel ledit premier conduit de lumière (36) peut être inséré dans ledit cathéter creux (31) après avoir disposé une gaine dudit cathéter creux dans ledit tissu.

3. Système selon l'une quelconque des revendications 1 ou 2, dans lequel ledit deuxième conduit de lumière (38) est une fibre à plusieurs utilisations.

4. Système selon l'une quelconque des revendications 1 à 3, comprenant en outre un dispositif d'alimentation destiné à fournir un agent par le biais dudit cathéter creux (31) audit tissu (8).

5. Système selon l'une quelconque des revendications 1 à 4, comprenant en outre un dispositif d'alimentation destiné à fournir un agent par le biais dudit cathéter creux (31) audit tissu (8) et, dans lequel ledit cathéter creux (31) est doté d'un orifice (34) pour fournir ledit agent.

6. Système selon l'une quelconque des revendications 1 à 5,
comprenant un deuxième adaptateur ayant un autre connecteur optique prévu pour une connexion proximale audit deuxième conduit de lumière (38) pour connecter de manière libérable l'extrémité proximale dudit corps d'adaptateur audit autre connecteur optique disposé de manière proximale au niveau dudit connecteur optique (37).

7. Système selon l'une quelconque des revendications 1 à 6, dans lequel ledit élément de montage de connecteur optique au niveau de l'extrémité proximale est configuré pour un montage direct dudit connecteur optique (37) sur le corps d'adaptateur.

8. Système selon l'une quelconque des revendications 1 à 7, dans lequel ledit élément de montage de cathéter au niveau de l'extrémité distale du corps d'adaptateur (32) comprend une surface de montage pour un engagement de manière étanche avec ledit cathéter.

9. Système selon la revendication 8, dans lequel ladite surface de montage est une surface inclinée interne dimensionnée pour s'engager par emboîtement et fermer de façon étanche une surface inclinée externe d'un connecteur Luer dans ledit cathéter, ou dans lequel ladite surface de montage est filetée pour s'engager par emboîtement et fermer de façon étanche une surface filetée comprise dans ledit cathéter.

10. Système selon l'une quelconque des revendications 1 à 9, dans lequel ledit moyen d'étanchéité est un joint à compression radiale ayant un trou central destiné à recevoir le conduit de lumière dans celui-ci, positionné dans le corps d'adaptateur le long de la lumière axiale à une certaine distance par rapport à l'extrémité proximale, dans lequel le joint peut être comprimé radialement vers l'extérieur par ledit conduit de lumière pour permettre ladite étanchéité, ou dans lequel ledit moyen d'étanchéité comprend un moyen de fixation de sorte que ledit conduit de lumière soit fixé de manière étanche dans ladite lumière axiale par ledit moyen de fixation, comme de la colle, et/ou dans lequel ledit élément de montage de cathéter est destiné à monter un dispositif de cathéter intraveineux transcutané, ou dans lequel ledit élément de montage de cathéter est destiné à monter un dispositif de cathéter intraveineux transcutané, et dans lequel ledit cathéter a une longueur d'approximativement moins de 10 cm, et/ou dans lequel ledit adaptateur lorsqu'il est monté sur ledit cathéter ferme de manière étanche ladite lumière vis-à-vis d'une connexion de fluide provenant dudit côté proximal dudit adaptateur et permet une connexion lumineuse à partir de ladite extrémité proximale dudit adaptateur vers ladite lumière par le biais dudit conduit de lumière.

11. Système selon l'une quelconque des revendications 1 à 10, comprenant en outre une aiguille qui peut être insérée et retirée dans le cathéter creux pour disposer ledit cathéter sur ledit tissu lorsque ledit adaptateur est déconnecté dudit cathéter, de sorte que ledit conduit de lumière puisse être inséré à travers le cathéter ou l'aiguille en direction dudit tissu (8).

12. Système selon l'une quelconque des revendications 1 à 11, comprenant en outre le fait que ladite extrémité distale dudit premier conduit de lumière est radialement en contact avec la paroi intérieure d'une lumière s'étendant axialement dudit cathéter.

13. Procédé de montage d'un adaptateur d'extrémité proximale d'un cathéter, l'adaptateur d'extrémité proximale ayant un corps d'adaptateur (32), ayant des extrémités proximale et distale, définissant une lumière axiale s'étendant entre les extrémités proximale et distale définissant un trajet optique axial entre les extrémités proximale et distale du corps d'adaptateur, et un élément de montage de cathéter au niveau de l'extrémité distale du corps d'adaptateur (32) pour monter un cathéter sur le corps d'adaptateur, un élément de montage de connecteur optique au niveau de l'extrémité proximale pour monter un connecteur optique (37) sur le corps d'adaptateur, et un premier conduit de lumière (36) ayant des extrémités proximale et distale, dans lequel ledit premier conduit de lumière (36) est contenu de manière étanche par un moyen d'étanchéité dans ladite lumière axiale (33), ledit procédé comprenant les étapes suivantes consistant à :
positionner ledit premier conduit de lumière (36) avec l'extrémité proximale de celui-ci dans le corps d'adaptateur (32),
monter l'adaptateur d'extrémité proximale sur un boîtier de cathéter par ledit élément de montage de cathéter, pour fournir ledit premier conduit de lumière dans une lumière du cathéter,
monter un connecteur optique (37) sur ledit élément de montage de connecteur optique dudit corps d'adaptateur (32), en couplant de cette manière une extrémité distale d'un deuxième conduit de lumière optique (38) compris dans ledit connecteur optique (37) sur l'extrémité proximale dudit premier conduit de lumière (36), donc
fournir un trajet optique par le biais dudit adaptateur.
